# EUROPEAN PATENT SPECIFICATION

(11) **EP 1 872 794 B1**
(45) Date of publication and mention of the grant of the patent: **12.12.2012**
(21) Application number: 06731229.8
(22) Date of filing: 30.03.2006
(51) Int. Cl.: A61K 9/00, A61K 31/7052, A61K 31/7056, A61K 39/39

(54) **NASAL VACCINE**
NASALE VAKZINE
VACCIN NASAL

(30) Priority: 01.04.2005 JP 2005106891; 01.04.2005 JP 2005106894
(43) Date of publication of application: 02.01.2008
(73) Proprietor: JAPAN as represented by DIRECTOR GENERAL OF NATIONAL INSTITUTE OF INFECTIOUS DISEASES, Tokyo 162-8640 (JP); Riken, Wakou-shi, Saitama 351-0198 (JP)
(72) Inventor: MORI, Kenji c/o Riken Yokohama Institute, Yokohama-shi, Kanagawa 2300045 (JP); FUHSHUKU, Ken-ichi c/o Riken Yokohama Institute, Yokohama-shi, Kanagawa 2300045 (JP); TASHIRO, Takuya c/o Riken Yokohama Institute, Yokohama-shi, Kanagawa 2300045 (JP); TANIGUCHI, Masaru c/o Riken Yokohama Institute, Yokohama-shi, Kanagawa 2300045 (JP); SEINO, Ken-ichiro c/o Riken Yokohama Institute, Yokohama-shi, Kanagawa 2300045 (JP); HASEGAWA, Hideki Nat. Inst. Infectious Diseases, Tokyo 1628640 (JP)
(74) Representative: Duckworth, Timothy John
(86) International application number: PCT/JP2006/307281
(87) International publication number: WO 2006/107097

(56) References cited:
- EP-A- 1 867 656
- WO-A1-98/44928
- WO-A1-03/016326
- JP-A- 09 315 980
- KO SUNG YOUL ET AL: "alpha-Galactosyl ceramide (alpha GalCer) can act as a nasal vaccine adjuvant" FASEB JOURNAL, vol. 19, no. 4, Suppl. S, Part 1, March 2005 (2005-03), page A950, XP008106914 & EXPERIMENTAL BIOLOGY 2005 MEETING/35TH INTERNATIONAL CONGRESS OF PHYSIOLOGICAL SCIENCES; SAN DIEGO, CA, USA; MARCH 31 -APRIL 06, 2005 ISSN: 0892-6638
- BRADNEY C.P. ET AL.: 'Cytokines as Adjuvants for the Induction of Anti-Human Immunodeficiency Virus Peptide Immunoglobulin G (IgG) and IgA Antibodies in Serum and Mucosal Secretions after Nasal Immunization' JOURNAL OF VIROLOGY vol. 76, no. 2, 2002, pages 517 - 524, XP003005884
- GONZALEZ-ASEQUINOLAZA G. ET AL.: 'Natural Killer T Cell Ligand alpha-Galactosylceramide Enhances Protective Immunity Induced by Malaria Vaccines' J. EXP. MED. vol. 195, no. 5, 2002, pages 617 - 624, XP003005885
- TSUJI M.: 'Malaria no Atarashii Men'eki Kijo-NKT Saibo no Kanyo-' KANSEN ENSHO MEN'EKI vol. 32-4, 2002, pages 232 - 239, XP003005886
- KAWANO T. ET AL.: 'CD1d-Restricted and TCR-Mediated Activation of Valpha14 NKT Cells by Glycosylceramides' SCIENCE vol. 278, 1997, pages 1626 - 1629, XP002175772
- NAKANISHI K.: 'IL-18 ni yoru Th1/Th2 Men'eki Oto no Chosetsu' IGAKU NO AYUMI vol. 207, no. 4, 2003, pages 237 - 243, XP003005887

## Description

### Technical Field

The present invention relates to a nasal vaccine, and particularly, a nasal vaccine containing an communogen and the compound having an NKT cell-activating action as an active ingredient. Specifically, the present invention relates to a vaccine capable of intranasally inducing an increase in antigen specific immunoglobulin observed in virus infection, pollinosis and the like.

### Background Art

While vaccines for subcutaneous injection are still employed at present, considering that many viruses invade into the body through the upper respiratory tract, a more effective prophylactic method is induction of antiviral immunity into this moiety. With a vaccine for subcutaneous injection containing a virus surface protein component as an antigen, only IgG in blood stream rises and induction of topical immunity into the upper respiratory tract is impossible. While a method comprising intranasally administering an antigen together with an adjuvant has been developed (Tamura S, Ito Y, Asanuma H, Hirabayashi Y, Suzuki Y, Nagamine T, Aizawa C, Kurata T. Cross-protection against influenza virus infection afforded by trivalent inactivated vaccines inoculated intranasally with cholera toxin B subunit. J Immunol. 1992 Aug 1; 149(3):981-8.;Tamura S, Samegai Y, Kurata H, Nagamine T, Aizawa C, Kurata T. Protection against influenza virus infection by vaccine inoculated intranasally with cholera toxin B subunit. Vaccine. 1988 Oct; 6(5):409-13.;Tamura S, Yamanaka A, Shimohara M, Tomita T, Komase K, Tsuda Y, Suzuki Y, Nagamine T, Kawahara K, Danbara H, et al. Synergistic action of cholera toxin B subunit (and Escherichia coli heat-labile toxin B subunit) and a trace amount of cholera whole toxin as an adjuvant for nasal influenza vaccine. Vaccine. 1994 Apr;12(5):419-26.), a safe and effective adjuvant has not been found yet. Moreover, while clinical application of a method including intranasal administration of cold acclimated virus has recently been started in the US (Jin H, Lu B, Zhou H, Ma C, Zhao J, Yang CF, Kemble G, Greenberg H. Multiple amino acid residues confer temperature sensitivity to human influenza virus vaccine strains (FluMist) derived from cold-adapted A/Ann Arbor/6/60. Virology. 2003 Feb 1;306(1) :18-24.), administration to infants and the elderly has been excluded, with a remaining doubt in the safety.

Meanwhile, as a new lymphocyte population having an NK cell receptor and a T cell receptor, which shows different characteristics from other lymphoid lineage (T, B, NK cells), NKT cells have been identified. One of the functions characterizing the NKT cells is, for example, a function to recognize, as an antigen, a glycolipid (α-galactosylceramide) presented on CD1d belonging to the major histocompatible complex (MHC) class I molecule, and abundantly produce cytokine such as IL-4 and the like. Taking note of such function of the NKT cells, NKT cell activators and therapeutic agents of autoimmune diseases, which contain α-galactosylceramide as an active ingredient have been proposed (WO 98/044928). In addition, it has been reported that a certain different kind of glycolipid can treat autoimmune diseases by effectively expressing the immunoregulatory function in NKT cells (WO 03/016326). However, there is no report showing that an immune response, particularly, intranasal immune response, can be induced and enhanced by administering an NKT cell activator together with an immunogen to patients.

EP-A-1867656 describes compounds having a cyclic structure, which are useful for the prophylaxis or treatment of autoimmune diseases.

Ko Sung Youl et al: Faseb Journal, vol. 19, No. 4, Suppl S., Part 1, March 2005, Page A950, describes that α-Galactosyl ceramide (α GalCer) can act as a nasal vaccine adjuvant.

### Disclosure of the Invention

The present invention aims at providing a nasal vaccine capable of inducing an intranasally effective immune reaction. Specifically, the present invention aims at providing a nasal vaccine capable of inducing an increase in the antigen specific antibody titer represented by an increase in IgA and IgG. Moreover, the present invention aims at providing use of the nasal vaccine as a vaccine for virus infection or treatment of pollinosis.

In view of the above-mentioned problems, the present inventors have conducted intensive studies and observed an intranasal increase in antigen specific IgA, an increase in antigen specific IgG in blood, and a remarkable decrease in virus titer in an intranasal virus challenge by intranasally administering simultaneously the compound having an NKT cell-activating action during administration of an immunogen, and found that intranasally effective antiviral immunity can be induced by activation of NKT cell lineage, which resulted in the completion of the present invention. Accordingly, the present invention provides the following.
[1] A compound having an NKT cell-activating action represented by the following formula (I): 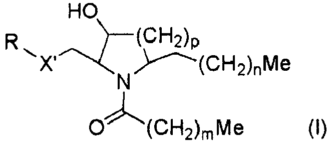 wherein R is an aldopyranose residue, X' is an oxygen atom, a sulfur atom or -NH-, m is an integer of 0 to 26, n is an integer of 0 to 16, p is an integer of 0 to 4, and Me is a methyl group, in combination with an immunogen in an amount effective for stimulating an immune response, for use as a nasal vaccine.
[2] Compound for use according to the above-mentioned [1], wherein the immunogen is derived from a pathogenic microorganism. wherein the immunogen is derived from a pathogenic microorganism.
[3] Compound for use according to the above-mentioned [2], wherein the pathogenic microorganism is a virus.
[4] Compound for use according to the above-mentioned [2], wherein the pathogenic microorganism is at least one kind selected from the group consisting of influenza virus, avian influenza virus, severe acute respiratory syndrome (SARS) virus, acquired immunodeficiency syndrome (AIDS) virus and *Streptococcus pneumoniae.*
[5] Compound for use according to the above-mentioned [1], wherein the immunogen is derived from pollen.
[6] Compound for use according to the above-mentioned [1] to [5], wherein R is α-D-galactopyranosyl.
[7] Compound for use according to the above-mentioned [1] to [6] wherein p is 0 or 1.
[8] Compound for use according to the above-mentioned [1] to [7], wherein n is 11 or 12.
[9] Compound for use according to the above-mentioned [1] to [8], wherein m is 24.
[10] Compound for use according to the above-mentioned [1] to [9], wherein X' is an oxygen atom.
[11] A combination of (i) an immunogen in an amount effective for stimulating an immune response and (ii) an effective amount of a compound having an NKT cell-activating action represented by the following formula (I): 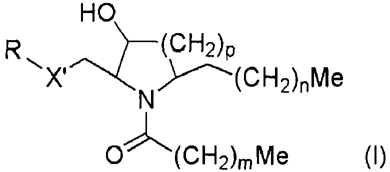 wherein R is an aldopyranose residue, X' is an oxygen atom, a sulfur atom or -NH-, m is an integer of 0 to 26, n is an integer of 0 to 16, p is an integer of 0 to 4, and Me is a methyl group, for use in inducing an intranasal immune response in a subject.
[12] The combination of the above-mentioned [11], wherein the immunogen is derived from a pathogenic microorganism.
[13] The combination of the above-mentioned [12], wherein the pathogenic microorganism is a virus.
[14] The combination of the above-mentioned [13], wherein the pathogenic microorganism is at least one kind selected from the group consisting of influenza virus, avian influenza virus, severe acute respiratory syndrome (SARS) virus, acquired immunodeficiency syndrome (AIDS) virus and *Streptococcus pneumoniae.*
[15] The combination of the above-mentioned [11], wherein the immunogen is derived from pollen.
[16] The combination of the above-mentioned [11] to [15] wherein R is α-D-galactopyranosyl.
[17] The combination of the above-mentioned [11] to [10], wherein p is 0 or 1.
[18] The combination of the above-mentioned [11] to [17], wherein n is 11 or 12.
[19] The combination of the above-mentioned [11] to [10], wherein m is 24.
[20] The combination of the above-mentioned [11] to [19], wherein X' is an oxygen atom.
[21] Use of a compound having an NKT cell-activating action represented by the following formula (I): 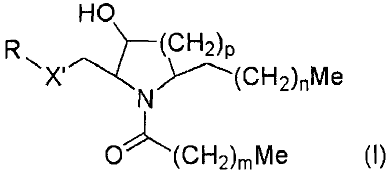 wherein R is an aldopyranose residue, X' is an oxygen atom, a sulfur atom or -NH-, m is an integer of 0 to 26, n is an integer of 0 to 16, p is an integer of 0 to 4, and Me is a methyl group and an immunogen for the production of a nasal vaccine.

### Brief Description of the Drawings

Fig. 1 shows a series of schedule of HA vaccine administration and virus challenge. (A):RCAI-8, (B):RCAI-0
Fig. 2 shows the measurement results of antibody titers of intranasal antigen specific IgA and antigen specific IgG in blood on nasal administration of the vaccine of the present invention. Furthermore, the Figure shows the measurement results of the virus titer in nasal lavage fluid of mouse which received an administration of the vaccine of the present invention and the virus challenge. (A):RCAI-8, (B):RCAI-0
Fig. 3 shows the effect of the vaccine of the present invention on the intranasal antigen specific IgA antibody titer, antigen specific IgG antibody titer in blood and the virus titer of intranasal washing of mouse subjected to virus challenge.
Fig. 4 shows the effect of the vaccine of the present invention on the survival rate after H5N1 virus challenge.

### Best Mode for Embodying the Invention

In the present invention, the "nasal vaccine" means a vaccine capable of inducing an intranasal immune response by transnasal vaccination. That is, the vaccination method is not particularly limited as long as it can induce an immune mechanism in the topical mucous membrane of the respiratory tract (particularly upper respiratory tract), which is an infection route of immunogen such as virus and the like. Examples of the method include spraying, swabbing, dropwise addition and the like.

The nasal vaccine of the present invention is characterized in that it contains an immunogen in an amount effective for inducing an immune response, particularly intranasal immune response, and a compound having an NKT cell-activating action represented by the formula (I).

The compound represented by the following formula (I) (hereinafter compound I): 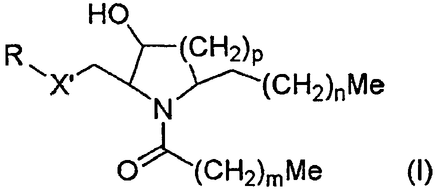 wherein R is an aldopyranose residue, X' is an oxygen atom, a sulfur atom or -NH-, m is an integer of 0 to 26, n is an integer of 0 to 16, p is an integer of 0 to 4, and Me is a methyl group.

While compound I includes structural isomers of α form and β form, it may be in any form of an α form, a β form and a mixture thereof. From the aspect of pharmacological effect, an α form is preferable. Specifically, compounds represented by the following formulas (2) to (5) (hereinafter compounds 2 to 5) are preferable. Of these, compound 2 and compound 4 are more preferable.

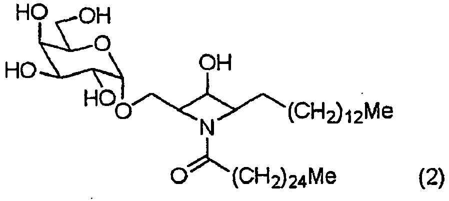

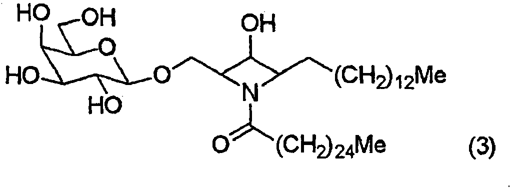

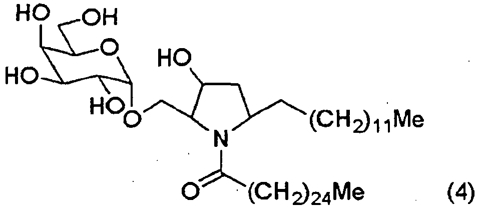

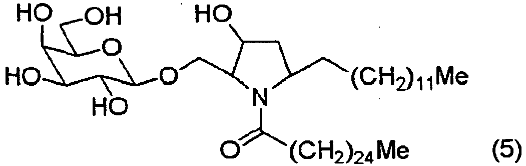

Compound I can be produced according to various methods. For example, it can be produced according to the method of <Scheme 1> described below.

Including the formula (I), the definition of each symbol in the following Scheme is as follows.

R is an aldopyranose residue, R' is an aldopyranose residue wherein the hydroxyl group is protected. The aldopyranose residue means a residue obtained by excluding the reducing terminal hydroxyl group from aldopyranose. As the aldopyranose residue, for example, α-D-galactopyranosyl, α-D-glucopyranosyl, β-D-galactopyranosyl, β-D-glucopyranosyl and the like can be mentioned. Of these, α-D-galactopyranosyl is preferable from the aspect of pharmacological effect. As the hydroxyl-protecting group, acyl group, t-butyldimethylsilyl (TBS) group, benzyl (Bn) group, p-methoxybenzyl (PMB) group and the like can be mentioned, and benzyl (Bn) group, p-methoxybenzyl (PMB) group are preferable.

The -OC(CH₂)ₘCH₃ group is a residue of fatty acid having 2 to 28 carbon atoms wherein terminal hydroxyl group has been removed therefrom. X' is an oxygen atom, a sulfur atom or -NH-group, and an oxygen atom is preferable.

m is an integer of 0 to 26, preferably 14 to 26, particularly preferably 24. n is an integer of 0 to 16, preferably 4 to 12, more preferably 10 to 12, particularly preferably 11 or 12. p is an integer of 0 to 4, preferably 0 to 2, particularly preferably 0 or 1.

The compounds represented by the formula (6) to the formula (11) are also referred to as compounds 6 to 11, respectively.

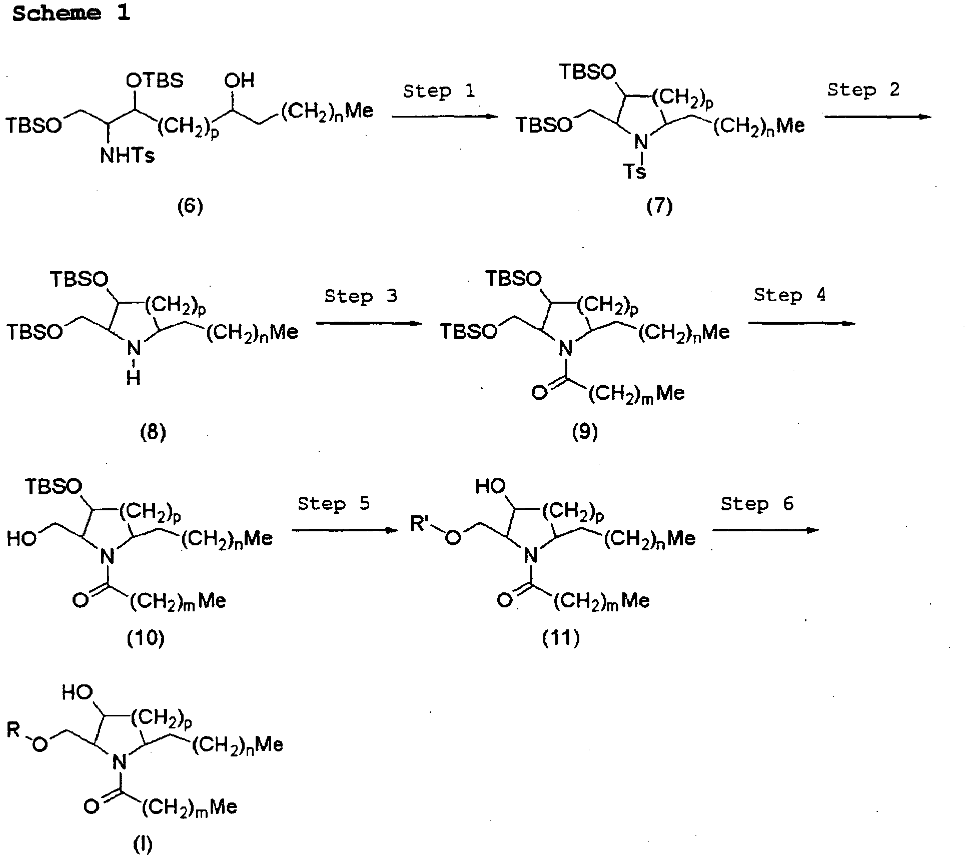

### (Step 1)

Step 1 is a step for intramolecularly cyclizing compound 6 to obtain compound 7. For this step, the method described in Tetrahedron Lett. 1995, 36, 7689 or J. Chem. Soc., Perkin Trans.1, 1997, 97 can be applied. Specifically, methanesulfonyl chloride is added in the presence of compound 6 and a base, and the mixture is reacted. As the base, pyridine, triethylamine, diisopropylethylamine and the like can be mentioned, and pyridine is preferable. When pyridine is used as a base, the amount of pyridine to be used is 2- to 50-fold volume, preferably 5- to 20-fold volume, relative to compound 6. The amount of the methanesulfonyl chloride to be used is generally 1.5 to 10 equivalents, preferably 2 to 8 equivalents, relative to compound 6. The reaction temperature is generally -20°C to room temperature, preferably 0 to 4°C, and the reaction time is generally 12 to 72 hr, preferably 18 to 48 hr. The above-mentioned reaction can be performed in the presence of a solvent as necessary. As the solvent, any solvent can be used as long as it does not inhibit this reaction. For example, halogen solvents (e.g., dichloromethane, chloroform) can be mentioned. After completion of the reaction, the reaction mixture is diluted with water, and extracted with a solvent such as diethyl ether and the like. The obtained organic layer is washed with saturated aqueous copper sulfate solution, water, saturated brine and the like, and dried over anhydrous magnesium sulfate and the like.

Then, the obtained crude product is dissolved in a solvent, sodium hydride is added thereto, and the mixture is stirred. The amount of sodium hydride to be used is generally 2 to 6 equivalents, preferably 3 to 4 equivalents, relative to compound 6. The reaction temperature is generally 0 to 60°C, preferably room temperature, and the reaction time is generally 12 to 72 hr, preferably 24 to 48 hr. As the solvent, any solvent can be used as long as it does not inhibit this reaction. For example, ether solvents can be mentioned, and tetrahydrofuran is particularly preferable. The amount of the solvent to be used is generally 5- to 50-fold volume, preferably 10- to 20-fold volume, relative to the crude product. After completion of the reaction, the reaction mixture is diluted with water and saturated aqueous ammonium chloride solution, and extracted with a solvent such as diethyl ether and the like. The obtained organic layer is washed with saturated brine and the like, dried over anhydrous magnesium sulfate and the like, and filtrated. The filtrate is concentrated under reduced pressure, and the residue is purified by column chromatography to give compound 7 in a high yield. Compound 6 can be obtained by subjecting an aldehyde and an alkyne as starting materials to a coupling reaction, followed by a number of steps.

### (Step 2)

Step 2 is a step for detosylating (Ts) compound 7 to obtain compound 8. This step is performed in a solvent. For example, sodium naphthalenide is added to a solution of compound 7 to allow a reaction. The amount of the sodium naphthalenide to be used is 5 to 50 equivalents, preferably 8 to 20 equivalents, relative to compound 7. The reaction temperature is generally -78°C to -20°C, preferably -78°C to - 60°C, and the reaction time is generally 1 to 4 hr, preferably 1 to 2 hr. Sodium naphthalenide can be prepared according to a conventionally known method. As the solvent, for example, ether solvents can be mentioned, and 1,2-dimethoxyethane is particularly preferable. The amount of the solvent to be used is generally 5- to 50-fold volume, preferably 10- to 20-fold volume, relative to compound 7. After completion of the reaction, the reaction mixture is diluted with water, and extracted with a solvent such as chloroform and the like. The obtained organic layer is washed with saturated brine and the like, dried over anhydrous magnesium sulfate and the like, and filtrated. The filtrate is concentrated under reduced pressure, and the residue is purified by column chromatography to give compound 8 in a high yield.

### (Step 3)

Step 3 is a step for acylating -NH- of compound 8 to obtain compound 9. This step is performed in a solvent. For example, a base, a condensing agent and a fatty acid having 2 to 28 carbon atoms are added to a solution of compound 8, and the mixture is reacted. As the base, the aforementioned base can be recited and, of these, diisopropylethylamine is preferable. As the condensing agent, a conventionally known condensing agent can be used. For example, carbodiimides such as 1,3-diisopropylcarbodiimide (DIC), 1-ethyl-3-(3-dimethylaminopropyl)carbodiimide (EDC) hydrochloride and the like can be mentioned, and EDC hydrochloride is more preferable. As the fatty acid, higher fatty acid is preferable, and saturated fatty acid having 16 to 28 carbon atoms is more preferable. Specifically, palmitic acid, stearic acid, cerotic acid and the like can be mentioned and, of these, cerotic acid is particularly preferable. The amount of the base to be used is 2- to 12-fold volume, preferably 2- to 4-fold volume, relative to compound 8. The amount of the condensing agent to be used is 1 to 6 equivalents, preferably 1 to 2 equivalents, relative to compound 8. The amount of fatty acid to be used is 1 to 6 equivalents, preferably 1 to 2 equivalents, relative to compound 8. Where necessary, a catalytic amount of 4-(dimethylamino)pyridine and the like can be added. The reaction temperature is generally 0°C to heating under reflux conditions, preferably room temperature, and the reaction time is generally 24 to 96 hr, preferably 48 to 72 hr. As the solvent, for example, halogen solvents can be mentioned, and dichloromethane is particularly preferable. The amount of the solvent to be used is generally 5- to 50-fold volume, preferably 10- to 20-fold volume, relative to compound 8. After completion of the reaction, the reaction mixture is diluted with water, and extracted with a solvent such as diethyl ether and the like. The obtained organic layer is washed with saturated brine and the like, dried over anhydrous magnesium sulfate and the like, and filtrated. The filtrate is concentrated under reduced pressure, and the residue is purified by column chromatography to give compound 9 in a high yield.

### (Step 4)

Step 4 is a step for eliminating the endprotecting group bonded to the 2-position in the ring structure of compound 9 to obtain compound 10. This step is performed in a solvent. For example, an acid such as trifluoromethanesulfonic acid and the like is added to a solution of compound 9 to allow reaction. The amount of the acid to be used is a catalytic amount to 10-fold volume, preferably 1- to 2-fold volume, relative to compound 9. The reaction temperature is generally -20°C to room temperature, preferably room temperature, and the reaction time is generally 2 to 12 hr, preferably 2 to 4 hr. As the solvent, for example, ether solvents can be mentioned, and tetrahydrofuran is particularly preferable. The amount of the solvent to be used is generally 5- to 100-fold volume, preferably 10- to 50-fold volume, relative to compound 9. After completion of the reaction, the reaction mixture is neutralized with a basic aqueous solution such as aqueous sodium hydroxide solution and the like, and extracted with a solvent such as diethyl ether and the like. The obtained organic layer is washed with saturated aqueous sodium hydrogencarbonate solution, saturated brine and the like, dried over anhydrous magnesium sulfate and the like, and filtrated. The filtrate is concentrated under reduced pressure, and the residue is purified by column chromatography to give compound 10 in a high yield.

### (Step 5)

Step 5 is a step for aldopyranosylating and deprotecting compound 10 to obtain compound 11. This step is performed in a solvent. For example, aldopyranosyl halide is added to a solution of compound 10 in the presence of tin chloride, silver perchlorate and a dehydrating agent (e.g., molecular sieves), and the mixture is reacted. The amount of tin chloride to be used is 2 to 4 equivalents, preferably 3 to 4 equivalents, relative compound 10. The amount of silver perchlorate to be used is 2 to 4 equivalents, preferably 3 to 4 equivalents, relative to compound 10. The amount of the dehydrating agent to be used is 2- to 4-fold weight, preferably 3- to 4-fold weight, relative to compound 10. As aldopyranosyl halide, an aldopyranosyl halide wherein hydroxyl groups at the 2, 3, 4, 6-positions are protected by benzyl (Bn) group is preferable. As the halogen, fluorine atom is preferable. The amount of the aldopyranosyl halide to be used is 2 to 4 equivalents, preferably 2 to 3 equivalents, relative to compound 10. The reaction temperature is generally -20°C to room temperature, and the reaction time is generally 2 to 12 hr, preferably 2 to 4 hr. As the solvent, for example, ether solvents can be mentioned, and tetrahydrofuran is particularly preferable. The amount of the solvent to be used is generally 10- to 100-fold volume, preferably 20- to 50-fold volume, relative to compound 10. After completion of the reaction, the reaction mixture is filtrated using silica gel and the like. The filtrate is washed with saturated brine and the like, dried over anhydrous magnesium sulfate and the like, and filtrated. The filtrate is concentrated under reduced pressure, and the residue is purified by column chromatography to give two fractions (low polar fraction, high polar fraction).

Then, the obtained two fractions (low polar fraction, high polar fraction) are respectively dissolved in an ether solvent such as tetrahydrofuran and the like, tetrabutylammonium fluoride is added to this solution, and the mixture is stirred at room temperature for about 12 to 48 hr. After completion of the reaction, the reaction mixture is diluted with water, and extracted with diethyl ether and the like. The obtained organic layer is washed with saturated brine and the like, dried over anhydrous magnesium sulfate and the like, and filtrated. The filtrate is concentrated under reduced pressure, and the residue is purified by column chromatography to give compound 11 wherein the hydroxyl groups on the 2, 3, 4, 6-positions of the aldopyranose residue are protected by benzyl (Bn) group. While compound 11 includes an α form and a β form, the α form and the β form can be efficiently separated and purified by separating into two fractions (e.g., low polar fraction, high polar fraction) and subjecting each fraction to this operation. Alternatively, the α form and the β form of compound 11 can be separated and purified using solvents having different polarity during column chromatography.

### (Step 6)

Step 6 is a step for deprotecting compound 11 to obtain compound I. This step is performed in a solvent. For example, a catalyst of palladium hydroxide on carbon is added to a solution of compound 11, and the mixture is stirred under a hydrogen atmosphere at room temperature for about 12 to 24 hr. A catalytic amount of the palladium hydroxide on carbon catalyst relative to compound 11 is generally sufficient to be used. As the solvent, a mixed solvent of an alcohol solvent and a halogen solvent is preferable, and a mixed solvent of ethanol and chloroform is more preferable. The amount of the solvent to be used is generally 10- to 200-fold volume, preferably 50- to 150-fold volume, relative to compound 11. After completion of the reaction, the reaction mixture is filtrated with celite etc. and washed with the aforementioned solvent. The filtrate is concentrated under reduced pressure, and the residue is purified by column chromatography to give the object compound I in a high yield. Alternatively, the α form and the β form of compound I can be separated and purified using solvents having different polarity during column chromatography. In addition, the α form and the β form of compound I can be obtained using the α form and the β form of compound 11 isolated in step 5 as the starting materials.

<Scheme 2> described below is a step for ring-opening of epoxy compound represented by the formula (12) (hereinafter compound 12) to obtain a compound represented by the formula (13) (hereinafter compound 13).

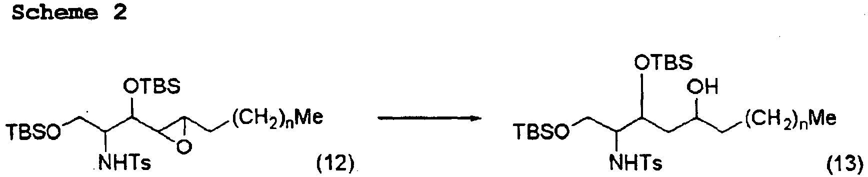

This step is performed in a solvent, where diisobutylaluminum hydride is added to a solution of compound 12 to allow reaction. The amount of diisobutylaluminum hydride to be used is generally 4 - 12 equivalents, preferably 4 - 8 equivalents, relative to compound 12. The reaction temperature is generally -78 to 0°C, and the reaction time is generally 3 to 6 hr. As the solvent, ether solvents are preferable, and tetrahydrofuran is particularly preferable. When a halogen solvent or hydrocarbon solvent is used, the above-mentioned reaction hardly proceeds. However, using tetrahydrofuran, the reaction can be specifically performed. The amount of the solvent to be used is generally 5- to 50-fold volume, preferably 10- to 20-fold volume, relative to compound 12. After completion of the reaction, saturated aqueous sodium potassium tartrate solution and the like are added to the reaction mixture, diluted with a solvent such as diethyl ether and the like and stirred and extracted with diethyl ether. The obtained organic layer is washed with saturated brine etc., dried over anhydrous magnesium sulfate and the like, and filtrated. The filtrate is concentrated under reduced pressure, and the residue is purified by column chromatography to give compound 13 in a high yield. An epoxy compound represented by the formula 12 can be produced according to Tetrahedron 1998, 54, 3141. Compound 13 is compound 6 wherein p=1. The reaction to obtain compound 6 by ring-opening of the corresponding epoxy compound can also be performed in the same manner.

Compound I may be a pharmaceutically acceptable non-toxic salt. For example, acid addition salts such as salts with an inorganic acid (hydrochloric acid, sulfuric acid, nitric acid, phosphoric acid etc.), salts with an organic acid (acetic acid, propionic acid, maleic acid, oleic acid, palmitic acid, citric acid, succinic acid, tartaric acid, fumaric acid, glutamic acid, pantothenic acid, lauryl sulfonic acid, methanesulfonic acid and phthalic acid etc.) and the like can be mentioned. Compound I may be a solvate (e.g., hydrate).

The compound of the present invention having an NKT cell-activating action can be contained in the nasal vaccine of the present invention in an amount effective for inducing an intranasal immune response to a particular immunogen. The compound may be mixed with the particular immunogen and administered as a single preparation. Alternatively, an immunogen and a compound having an NKT cell-activating action are separately prepared, made into preparations, and administered by mixing them when in use, or individually and almost simultaneously administered. Any method can be employed as long as it can induce an intranasal immune response.

The above-mentioned vaccine is provided in the form of a liquid or powder. A powder can be produced as a powder preparation by a method such as freeze-drying and the like. For intranasal administration such as intranasal spray, instillation, swabbing and the like, a liquid preparation is often suitable. However, a powder spray is also preferable. The vaccine of the present invention can contain a known stabilizer and a known preservative. As a stabilizer, about 0.1 - 0.2% of gelatin or dextran, 0.5 - 1% of sodium glutamate, or about 5% of lactose, about 2% of sorbitol and the like are used. As the preservative, about 0.01% of thimerosal and 0.1% of β-propiolactone are known.

The mixing ratio of the immunogen and the compound having an NKT cell-activating action in the vaccine of the present invention may be, for example, 1:0.5 - 1:5 (weight ratio). This range is an example of a general range, and a preferable ratio is determined and employed according to the kind of vaccine. The method necessary therefor is known to those of ordinary skill in the art.

As the subject for administration of the vaccine of the present invention, mammals such as human, monkey, mouse, rat, rabbit, cat, bovine, dog, horse, goat and the like and birds such as chicken and the like can be mentioned.

While the dose of the vaccine varies depending on the kind of immunogen, the age and body weight of the subject for administration, desired action and the like, it is, for example, 0.2 - 1.0 mL of vaccine is intranasally administered to the subject (adult, body weight about 60 kg) once to several times a day, preferably once a day (primary immunization). Generally, it is re-administered 2 - 3 weeks later in the same manner (additional immunization).

The immunogen usable for the vaccine of the present invention is not particularly limited as long as it can induce an intranasal immune response by intranasal administration together with a compound having an NKT cell-activating action. Moreover, it is not particularly limited as long as it is expected to afford an increase in the intranasal IgA antibody titer and an increase in the blood IgG antibody titer, as well as a decrease in the virus titer on intranasal virus challenge.

Representative immunogen includes protein and/or peptide and the like from, for example, viruses in human and animal (e.g., influenza virus, avian influenza virus, parainfluenza virus, adenovirus, SARS virus, AIDS virus, cytomegalovirus, hepatitis virus, Japanese encephalitis virus, measles virus and the like), bacteria (e.g., *Streptococcus pneumoniae, Neisseria meningitidis, Staphylococcus, Pseudomonas aeruginosa* and the like), fungi (e.g., *Cryptococcus·Aspergillus* and the like), protozoan (e.g., malaria and the like), other microorganisms and toxin, polysaccharide, cadaver of insect (e.g., mite and the like), pollen and the like.

These vaccines include various vaccines classified according to the production methods. That is, attenuated live vaccine, inactivated vaccine, component vaccine, vaccine based on DNA and the like are included. The vaccine based on DNA include a vaccine containing a DNA fragment incorporated into the vector such as plasmid, as well as a vaccine using ribozyme, antisense oligonucleotide and the like in combination. An immunogen component convenient for the effect of vaccine may be produced by a recombinant cell by applying the gene recombination technology. These vaccines may be a plain vaccine or a mixed vaccine. For example, an influenza vaccine may be a split vaccine containing haemagglutinin (HA), neuraminidase (NA), nuclear protein (NP), matrix protein (M) or a part thereof obtained by degradation and purification, using ether and a detergent, of a virus grown by a cell culture technique such as embryonated chicken egg, vero cell and the like, or by a gene recombination technology or chemical synthesis, and the like. A vaccine for pollinosis can be produced by using whole pollen or a part of peptide as an immunogen. As an immunogen derived from pollen, for example, Japanese cedar pollen and Cryj1, Cryj2 derived therefrom, Cupressaceae pollen and Chao1, Chao2 derived therefrom and the like can be mentioned. The peptide to be used in the present invention is not limited to those prepared by chemical synthesis and, for example, one collected from pollen or one obtained from a degraded product obtained by appropriately degrading a pollen allergen prepared by a recombinant DNA technique. For example, a DNA encoding the peptide may be prepared, inserted into an autoreplicatable vector to give a recombinant DNA, which is introduced into an appropriate host (e.g., *Escherichia coli, Bacillus subtilis,* actinomycetes, yeast and the like) to give a transformant, and the peptide may be harvested from a culture thereof. As the vector and the like, those generally used in the art can be utilized.

While the immunogen component (e.g., peptide) to be contained in the vaccine of the present invention shows a certain level of therapeutic or prophylactic effect even when administered in a comparatively crude state, it is generally purified before use. For purification, for example, a method conventionally used in the art for the purification of a peptide or protein, such as filtration, concentration, centrifugation, gel filtration chromatography, ion exchange chromatography, hydrophobic chromatography, adsorption chromatography, high performance liquid chromatography, affinity chromatography, gel electrophoresis, isoelectric focusing and the like and, where necessary, these methods may be combines as appropriate. According to the form of final use, purified peptide may be concentrated or freeze-dried to give a liquid or solid.

Vaccine can be produced according to a method generally employed in the art and using the above-mentioned immunogen.

### Examples

The present invention is explained in more detail in the following by referring to Examples, which are not to be construed as limitative to the scope of the present invention. In addition, unless otherwise specified, the reagents, apparatuses and materials to be used in the present invention are commercially available.

### Reference Example 1: Synthesis of compound having an NKT cell-activating action

The compound having an NKT cell-activating action can be synthesized according to the present Reference Examples.

### 1. Synthesis of compound represented by the formula (14) (hereinafter compound 14)

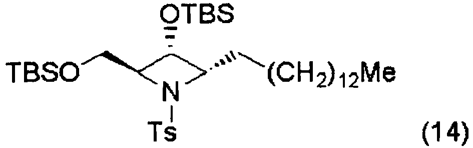

To a solution of a compound represented by the following formula (15) (hereinafter compound 15) (460 mg, 0.657 mmol) in absolute pyridine (5.0 ml) was added methanesulfonyl chloride (0.20 ml, 2.58 mmol) under ice-cooling, and the mixture was stirred at 4°C for 18 hr. The reaction mixture was diluted with water and extracted with diethyl ether. The combined organic layer was washed with saturated aqueous copper sulfate solution, water and saturated brine, and dried over anhydrous magnesium sulfate. After filtration, the filtrate was concentrated under reduced pressure to give a crude mesylated product. The crude mesylated product was dissolved in absolute tetrahydrofuran (5.0 ml), 60% sodium hydride (79.0 mg, 1.98 mmol) was added under ice-cooling, and the mixture was stirred at room temperature for 40 hr. The reaction mixture was diluted with water and saturated aqueous ammonium chloride solution and extracted with diethyl ether. The combined organic layer was washed with saturated brine, and dried over anhydrous magnesium sulfate. After filtration, the filtrate was concentrated under reduced pressure, and the residue was purified by silica gel column chromatography (25 g, hexane-ethyl acetate, 60:1 - 30:1) to give compound 14 (360 mg, 80%).

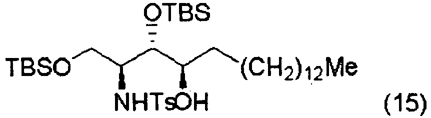

### 2. Synthesis of compound represented by the formula (16) (hereinafter compound 16)

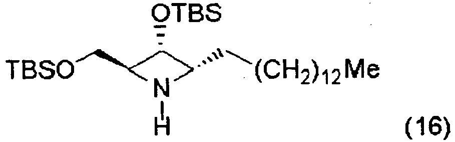

### (1) Preparation of sodium naphthalenide

Under an argon atmosphere, to a solution of naphthalene (516 mg, 4.03 mmol) in absolute 1,2-dimethoxyethane (5.0 ml) was added sodium (77.4 mg, 3.37 mmol), and the mixture was stirred at room temperature for 3 hr.

### (2) Detosylation

Under an argon atmosphere, to a solution of compound 14 (286 mg, 0.419 mmol) in absolute 1,2-dimethoxyethane (3.0 ml) was added dropwise the prepared sodium naphthalenide (5.0 ml) at -78°C. The reaction mixture was stirred for 90 min, diluted with water and extracted with chloroform. The combined organic layer was washed with saturated brine, and dried over anhydrous sodium sulfate. After filtration, the filtrate was concentrated under reduced pressure, and the residue was purified by silica gel column chromatography (15 g, chloroform -methanol,1:0 - 20:1) to give compound 16 (221 mg, 100%).

### 3. Synthesis of compound represented by the formula (17) (hereinafter compound 17)

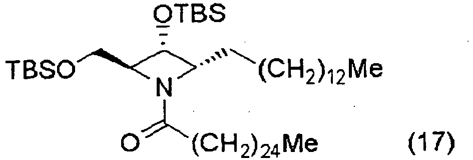

To a solution of compound 16 (129 mg, 0.244 mmol) in absolute dichloromethane (10.0 ml) were added diisopropylethylamine (0.30 ml, 1.72 mmol), cerotic acid (148 mg, 0.373 mmol), 1-ethyl-3-(3-dimethylaminopropyl)-carbodiimide hydrochloride (71.0 mg, 0.370 mmol) and a catalytic amount of 4-(dimethylamino)pyridine, and the mixture was stirred at room temperature for 62 hr. The reaction mixture was diluted with water and extracted with diethyl ether. The combined organic layer was washed with saturated brine, and dried over anhydrous magnesium sulfate. After filtration, the filtrate was concentrated under reduced pressure, and the residue was purified by silica gel column chromatography (5 g, hexane-ethyl acetate, 50:1 - 30:1) to give compound 17 (159 mg, 72%).

### 4. Synthesis of compound represented by the formula (18) (hereinafter compound 18)

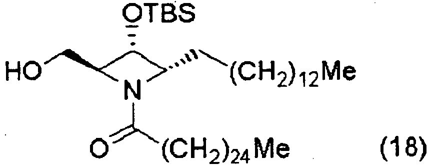

To a solution of compound 17 (64.2 mg, 70.8 µmol) in absolute tetrahydrofuran (3.0 ml) was added trifluoromethanesulfonic acid (10% aqueous solution, 1.0 ml) under ice-cooling, and the mixture was stirred at room temperature for 3 hr. The reaction mixture was neutralized with aqueous sodium hydroxide solution and extracted with diethyl ether. The combined organic layer was washed with saturated aqueous sodium hydrogencarbonate solution and saturated brine, and dried over anhydrous magnesium sulfate. After filtration, the filtrate was concentrated under reduced pressure, and the residue was purified by silica gel column chromatography (5 g, hexane-ethyl acetate, 50:1 - 4:1) to give compound 18 (55.7 mg, 99%).

### 5. Synthesis of compounds represented by the formulas (19) and (20) (hereinafter compound 19 and compound 20)

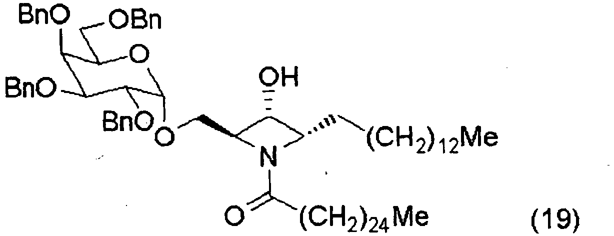

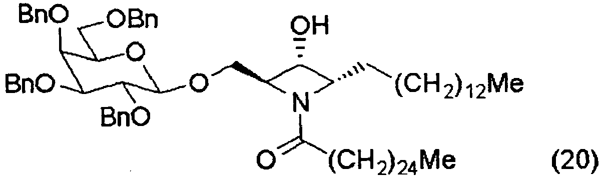

To a solution of compound 18 (127 mg, 0.160 mmol) in absolute tetrahydrofuran (5.0 ml) were added tin chloride (91.8 mg, 0.485 mmol), silver perchlorate (99.8 mg, 0.481 mmol) and molecular sieves 4A (300mg), and the mixture was stirred at room temperature for 90 min. Benzylglycosyl fluoride (210 mg, 0.387 mmol) was added at -20°C and the mixture was stirred and allowed to gradually warm to 10°C over 4 hr. After filtration of the reaction mixture using silica gel, the filtrate was washed with water and saturated brine, and dried over anhydrous magnesium sulfate. After filtration, the filtrate was concentrated under reduced pressure, and the residue was crudely fractionated by silica gel column chromatography (20 g, hexane-ethyl acetate, 1:0 - 6:1) into two fractions (low polar fraction 146 mg, high polar fraction 122 mg).

The obtained low polar fraction (146 mg) was dissolved in tetrahydrofuran (5.0 ml), tetrabutylammonium fluoride (1.0 M solution in tetrahydrofuran, 0.75 ml, 0.75 mmol) was added, and the mixture was stirred at room temperature for 15 hr. The reaction mixture was diluted with water and extracted with diethyl ether. The combined organic layer was washed with saturated brine, and dried over anhydrous magnesium sulfate. After filtration, the filtrate was concentrated under reduced pressure, and the residue was purified by silica gel column chromatography (20 g, hexane-ethyl acetate, 10:1 - 3:2) to give compound 19 (82.8 mg, two steps, 43%).

The obtained high polar fraction (122 mg) was dissolved in tetrahydrofuran (5.0 ml), tetrabutylammonium fluoride (1.0 M solution in tetrahydrofuran, 0.75 ml, 0.75 mmol) was added, and the mixture was stirred at room temperature for 15 hr. The reaction mixture was diluted with water and extracted with diethyl ether. The combined organic layer was washed with saturated brine, and dried over anhydrous magnesium sulfate. After filtration, the filtrate was concentrated under reduced pressure, and the residue was purified by silica gel column chromatography (20 g, hexane-ethyl acetate, 10:1 - 3:2) to give compound 20 (93.4 mg, two steps, 49%).

### 6. Synthesis of compound represented by the formula (21) (hereinafter compound 21)

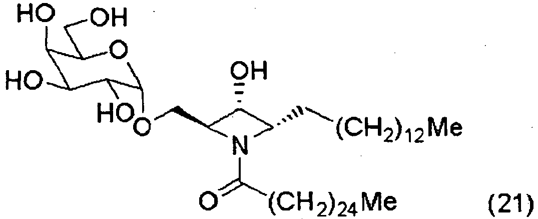

Under an argon atmosphere, to a solution of compound 19 (44.2 mg, 36.8 µmol) in a mixture of ethanol (3.0 ml) and chloroform (1.0 ml) was added 20% palladium hydroxide on carbon catalyst (5 mg) and, under a hydrogen atmosphere, the mixture was vigorously stirred at room temperature for 20 hr. The reaction mixture was filtered through celite, and washed with chloroform and methanol. The filtrate was concentrated under reduced pressure, and the residue was purified by silica gel column chromatography (3 g, chloroform -methanol, 20:1 - 10:1) to give compound 21 (12.7 mg, 41%).
¹H NMR (500 MHz, pyridine-d) δ 0.83-0.86 (6H, m), 1.14-2.60 (74H, m), 4.01 (0.40H, dd, J = 3.7, 11.0 Hz), 4.13 (0.60H, dd, J = 2.7, 10.3 Hz), 4.33-4.72 (8.40H, m), 4.81 (0.60H, dd, J = 4.6, 10.3 Hz), 5.06-5.20 (1H, m), 5.40 (0.60H, J = 3.7 Hz), 5.44 (0.40H, J = 3.7 Hz)

### 7. Synthesis of compound represented by the formula (22) (hereinafter compound 22)

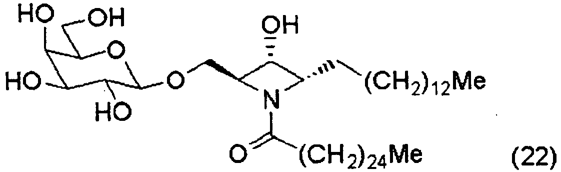

Under an argon atmosphere, to a solution of compound 20 (41.8 mg, 34.8 µmol) in a mixture of ethanol (3.0 ml) and chloroform (1.0 ml) was added 20% palladium hydroxide on carbon catalyst (5 mg), and under a hydrogen atmosphere, the mixture was vigorously stirred at room temperature for 20 hr. The reaction mixture was filtered through celite, and washed with chloroform and methanol. The filtrate was concentrated under reduced pressure, and the residue was purified by silica gel column chromatography (3 g, chloroform -methanol, 20:1 - 10:1) to give compound 22 (5.1 mg, 17%).
¹H NMR (500 MHz, CDCl₃-CD₃OD) δ 0.89 (6H, t, J = 6.8 Hz), 1.16-1.43 (64H, m), 1.54-1.67 (3.25H, m), 1.87 (0.25H, d, J = 14.2 Hz), 1.96 (0.75H, d, J = 13.7 Hz), 2.03-2.45 (3.75H, m), 3.31-4.33 (9H, m), 4.08 (0.25H, dd, J = 3.9, 10.5 Hz), 4.11 (0.75H, dd, J = 3.7, 10.5 Hz), 4.22 (0.25H, d, J = 7.6 Hz), 4.24 (0.75H, d, J = 7.6 Hz), 4.46 (0.75H, d, J = 4.9 Hz), 4.47 (0.25H, d, J = 4.9 Hz)
¹H NMR (500 MHz, pyridine-d) δ 0.83-0.86 (6H, m), 1.13-2.59 (74H, m), 4.02 (0.60H, m), 4.07-4.19 (2H, m), 4.40-4.50 (3.20H, m), 4.54-4.61 (2.60H, m), 4.68 (0.60H, m), 4.71 (0.60H, d, J = 11.0 Hz), 4.72 (0.40H, d, J = 10.7 Hz), 4.88 (0.40H, d, J = 7.8 Hz), 4.88 (0.60H, d, J = 7.8 Hz), 4.95 (0.40H, dd, J = 3.7, 6.8 Hz), 5.11 (0.60H, dd, J = 4.2, 6.8 Hz)

### 8. Synthesis of compound represented by the formula (23) (hereinafter compound 23)

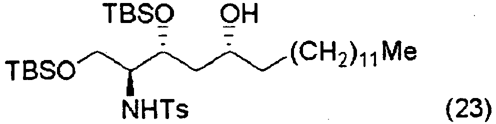

Under an argon atmosphere, to a solution of a compound represented by the following formula (24) (hereinafter compound 24) (540 mg, 0.773 mmol) in absolute tetrahydrofuran (10.0 ml) was added dropwise diisobutylaluminum hydride (0.95 M hexane solution, 4.2 ml, 3.99 mmol) at -78°C, and the mixture was allowed to gradually warm to 0°C over 3 hr. A saturated aqueous sodium potassium tartrate solution was added and the mixture was diluted with diethyl ether. The mixture was stirred at room temperature for 2 hr and extracted with diethyl ether. The combined organic layer was washed with saturated brine, and dried over anhydrous magnesium sulfate. After filtration, the filtrate was concentrated under reduced pressure, and the residue was purified by silica gel column chromatography (25 g, hexane-ethyl acetate, 30:1 - 15:1) to give compound 23 (462 mg, 85%).

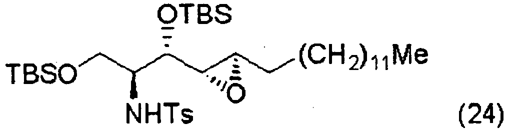

### 9. Synthesis of compound represented by the formula (25)

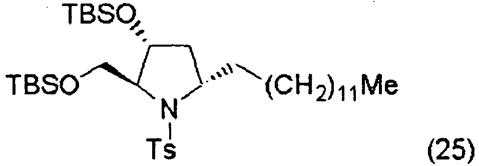

To a solution of compound 23 (1.024 g, 1.46 mmol) in absolute pyridine (10.0 ml) was added methanesulfonyl chloride (904 µl, 11.7 mmol) under ice-cooling, and the mixture was stirred at 4°C for 42 hr. The reaction mixture was diluted with water and extracted with diethyl ether. The combined organic layer was washed with saturated aqueous copper sulfate solution, water and saturated brine, and dried over anhydrous magnesium sulfate. After filtration, the filtrate was concentrated under reduced pressure to give a mesylated crude product. The mesylated crude product was dissolved in absolute tetrahydrofuran (10.0 ml), 60% sodium hydride (180 mg, 4.50 mmol) was added under ice-cooling, and the mixture was stirred at room temperature for 40 hr. The reaction mixture was diluted with water and extracted with diethyl ether. The combined organic layer was washed with saturated brine, and dried over anhydrous magnesium sulfate. After filtration, the filtrate was concentrated under reduced pressure, and the residue was purified by silica gel column chromatography (25 g, hexane-ethyl acetate, 30:1 - 20:1) to give compound 25 (973 mg, 97%).

### 10. Synthesis of compound represented by the formula (26) (hereinafter compound 26)

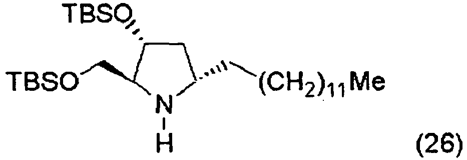

### (1) Preparation of sodium naphthalenide

Under an argon atmosphere, to a solution of naphthalene (1.86 g, 14.5 mmol) in absolute 1,2-dimethoxyethane (12.0 ml) was added sodium (267 mg, 11.6 mmol), and the mixture was stirred at room temperature for 3 hr.

### (2) Detosylation

Under an argon atmosphere, to a solution of compound 25 (484 mg, 0.693 mmol) in absolute 1,2-dimethoxyethane (3.0 ml) was added dropwise the prepared sodium naphthalenide (6.0 ml) at -78°C. The reaction mixture was stirred for 90 min, diluted with water, and extracted with chloroform. The combined organic layer was washed with saturated brine, and dried over anhydrous sodium sulfate. After filtration, the filtrate was concentrated under reduced pressure, and the residue was purified by silica gel column chromatography (25 g, chloroform -methanol, 1:0 - 20:1) to give compound 26 (349 mg, 93%).

### 11. Synthesis of compound represented by the formula (27) (hereinafter compound 27)

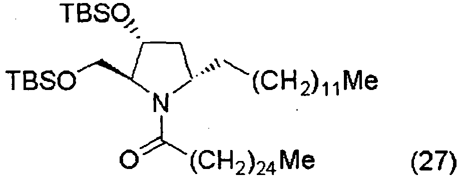

To a solution of compound 26 (132 mg, 0.250 mmol) in absolute dichloromethane (10.0 ml) were added diisopropylethylamine (0.30 ml, 1.72 mmol), cerotic acid (150 mg, 0.378 mmol), 1-ethyl-3-(3-dimethylaminopropyl)-carbodiimide hydrochloride (75.0 mg, 0.391 mmol), and a catalytic amount of 4-(dimethylamino)pyridine, and the mixture was stirred at room temperature for 42 hr. The reaction mixture was diluted with water and extracted with diethyl ether. The combined organic layer was washed with saturated brine, and dried over anhydrous magnesium sulfate. After filtration, the filtrate was concentrated under reduced pressure, and the residue was purified by silica gel column chromatography (20 g, hexane-ethyl acetate, 20:1 - 10:1) to give compound 27 (175 mg, 77%).

### 12. Synthesis of compound represented by the formula (28) (hereinafter compound 28)

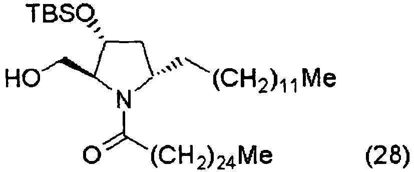

To a solution of compound 27 (86.2 mg, 95.1 µmol) in absolute tetrahydrofuran (3.0 ml) was added trifluoromethanesulfonic acid (10% aqueous solution, 1.0 ml) under ice-cooling, and the mixture was stirred at room temperature for 3 hr. The reaction mixture was neutralized with aqueous sodium hydroxide solution and extracted with diethyl ether. The combined organic layer was washed with saturated aqueous sodium hydrogencarbonate solution and saturated brine, and dried over anhydrous magnesium sulfate. After filtration, the filtrate was concentrated under reduced pressure, and the residue was purified by silica gel column chromatography (5 g, hexane-ethyl acetate, 50:1 - 4:1) to give compound 28 (69.0 mg, 92%).

### 13. Synthesis of compounds represented by the formulas (29) and (30) (hereinafter compound 29 and compound 30)

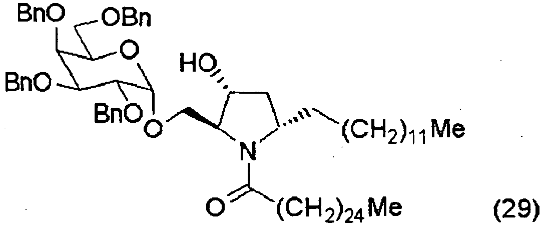

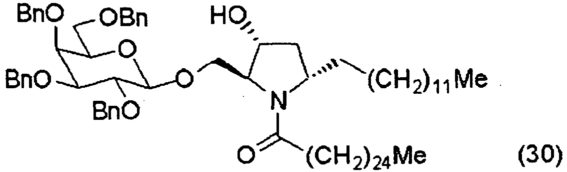

To a solution of compound 28 (114 mg, 0.114 mmol) in absolute tetrahydrofuran (5.0 ml) were added tin chloride (82.4 mg, 0.435 mmol), silver perchlorate (90.1 mg, 0.435 mmol) and molecular sieves 4A (300mg), and the mixture was stirred at room temperature for 90 min. Benzylglycosyl fluoride (189 mg, 0.348 mmol) was added at -20°C and the mixture was stirred, and the mixture was allowed to gradually warm to room temperature over 2 hr. After filtration of the reaction mixture using silica gel, the filtrate was washed with saturated brine, and dried over anhydrous magnesium sulfate. After filtration, the filtrate was concentrated under reduced pressure, and the residue was crudely fractionated by silica gel column chromatography (20 g, hexane-ethyl acetate, 10:1 - 6:1) into two fractions (low polar fraction 142 mg, high polar fraction 93mg).

The obtained low polar fraction (142 mg) was dissolved in tetrahydrofuran (4.0 ml), tetrabutylammonium fluoride (1.0 M solution in tetrahydrofuran, 0.50 ml, 0.50 mmol) was added, and the mixture was stirred at room temperature for 45 hr. The reaction mixture was diluted with water and extracted with diethyl ether. The combined organic layer was washed with saturated brine, and dried over anhydrous magnesium sulfate. After filtration, the filtrate was concentrated under reduced pressure, and the residue was purified by silica gel column chromatography (10 g, hexane-ethyl acetate, 10:1 - 3:1) to give compound 29 (84.4 mg, two steps, 49%).

The obtained high polar fraction (93 mg) was dissolved in tetrahydrofuran (4.0 ml), tetrabutylammonium fluoride (1.0 M solution in tetrahydrofuran, 0.50 ml, 0.50 mmol) was added, and the mixture was stirred at room temperature for 45 hr. The reaction mixture was diluted with water and extracted with diethyl ether. The combined organic layer was washed with saturated brine, and dried over anhydrous magnesium sulfate. After filtration, the filtrate was concentrated under reduced pressure, and the residue was purified by silica gel column chromatography (10 g, hexane-ethyl acetate, 8:1 - 3:1) to give compound 30 (32.0 mg, two steps, 19%).

### 14. Synthesis of compound represented by the formula (31) (hereinafter compound 31)

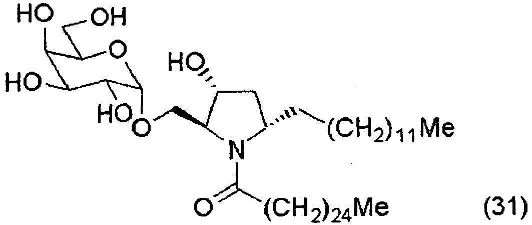

Under an argon atmosphere, to a solution of compound 29 (39.8 mg, 33.1 µmol) in a mixture of ethanol (3.0 ml) and chloroform (1.0 ml) was added 20% palladium hydroxide on carbon catalyst (5 mg) and, under a hydrogen atmosphere, the mixture was vigorously stirred at room temperature for 15 hr. The reaction mixture was filtered through celite, and washed with chloroform and methanol. The filtrate was concentrated under reduced pressure, and the residue was purified by silica gel column chromatography (3 g, chloroform -methanol, 12:1 - 8:1) to give compound 31 (26.5 mg, 95%).
¹H NMR (500 MHz, CDCl₃-CD₃OD) δ 0.88 (6H, t, J = 6.8 Hz), 1.15-1.38 (66H, m), 1.53-1.64 (2H, m), 1.84 (0.25H, d, J = 13.9 Hz), 1.97 (0.75H, d, J = 13.9 Hz), 2.00-2.40 (3H, m), 3.29 (0.75H, dd, J = 9.5, 9.5 Hz), 3.52 (0.25H, m), 3.63-3.98 (8.25H, m), 4.10 (0.75H, dd, J = 2.7, 8.8 Hz), 4.33 (0.75H, d, J = 4.9 Hz), 4.40 (0.25H, d, J = 4.6 Hz) 4.84 (0.25H, d, J = 3.9 Hz), 4.92 (0.75H, d, J = 3.9 Hz)
¹H NMR (500 MHz, pyridine-d) δ 0.83-0.87 (6H, m), 1.14-1.45 (64H, m), 1.76-1.95 (2.5H, m), 2.10 (0.5H, d, J = 13.4 Hz), 2.13-2.22 (0.5H, m), 2.20 (0.5H, d, J= 13.4 Hz), 2.45-2.64 (3.5H, m), 2.67-2.72 (0.5H, m), 3.75 (0.5H, dd, J = 3.4, 10.3 Hz), 3.91-3.97 (1H, m), 4.06-4.11 (1H, m), 4.25 (0.5H, dd, J= 3.2, 9.3 Hz), 4.31-4.49 (4.5H, m), 4.57 (0.5H, dd, J = 2.9, 9.0 Hz), 4.57 (1H, d, J = 2.9 Hz), 4.67 (0.5H, dd, J = 3.9, 10.0 Hz), 4.72 (0.5H, dd, J = 3.9, 10.0 Hz), 4.96 (0.5H, dd, J = 2.7, 7.8 Hz), 5.00 (0.5H, dd, J = 4.9, 4.9 Hz), 5.40 (0.5H, d, J = 3.9 Hz), 5.43 (0.5H, d, J = 3.7 Hz)

### 15. Synthesis of compound represented by the formula (32) (hereinafter compound 32)

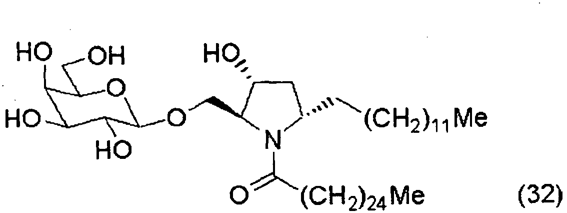

Under an argon atmosphere, to a solution of compound 30 (30.6 mg, 25.5 µmol) in a mixture of ethanol (3.0 ml) and chloroform (1.0 ml) was added 20% palladium hydroxide on carbon catalyst (5 mg) and, under a hydrogen atmosphere, the mixture was vigorously stirred at room temperature for 15 hr. The reaction mixture was filtered through celite, and washed with chloroform and methanol. The filtrate was concentrated under reduced pressure, and the residue was purified by silica gel column chromatography (2 g, chloroform -methanol, 10:1 - 6:1) to give compound 32 (13.6 mg, 64%).
¹H NMR (500 MHz, CDCl₃-CD₃OD) δ 0.89 (6H, t, J = 6.8 Hz), 1.16-1.43 (64H, m), 1.54-1.67 (3.25H, m), 1.87 (0.25H, d, J = 14.2 Hz), 1.96 (0.75H, d, J = 13.7 Hz), 2.03-2.45 (3.75H, m), 3.31-4.33 (9H, m), 4.08 (0.25H, dd, J = 3.9, 10.5 Hz), 4.11 (0.75H, dd, J = 3.7, 10.5 Hz), 4.22 (0.25H, d, J = 7.6 Hz), 4.24 (0.75H, d, J = 7.6 Hz), 4.46 (0.75H, d, J = 4.9 Hz), 4.47 (0.25H, d, J = 4.9 Hz)

### Example 1: Effect of the nasal vaccine of the present invention on influenza virus

### (1) Preparation of HA vaccine

HA vaccine was prepared according to the method of Davenport et al. (Davenport FM, Hennessy AV, Brandon FM, Webster RG, Barrett CD Jr, Lease GO. Comparisons Of Serologic And Febrile Responses In Humans To Vaccination With Influenza A Viruses Or Their Hemagglutinins. J Lab Clin Med. 1964 Jan;63:5-13.) from influenza virus A/PR8. Virus was cultured in the allantoic cavity of an embryonated chicken egg at day 10 - 11, purified from chorioallantoic fluid and partially lysed with ethyl ether to give HA vaccine. The vaccine contained total protein derived from virus particles, whose main component was HA molecule (about 30% of total protein). The virus acquired an ability to infect mouse by cultivating over 148 passages in ferret, 596 passages in mouse, and 73 passages in embryonated chicken egg at day 10.

### (2) Preparation of compound having NKT cell-activating action

As a compound having an NKT cell-activating action to be administered with virus antigen, RCAI-8 {compound (31) prepared in the above-mentioned Reference Example 1} and RCAI-0 (α-galactosylceramide; α-GalCer) were used. As RCAI-0, (2S,3S,4R)-1-(α-D-galactopyranosyloxy)-2-hexacosanoylamino-3,4-octadecanediol synthesized according to the method described in WO98/44928 was used.

### (3) Administration of HA vaccine and virus challenge

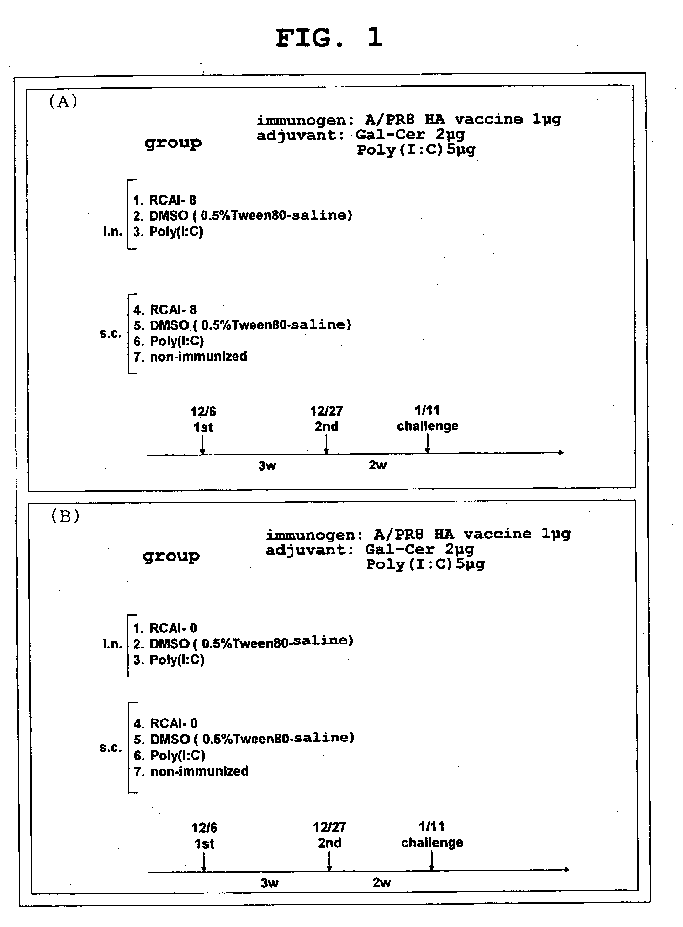

Five mice in each experiment group were anesthetized with diethyl ether, 0.5% Tween 80-saline solution (5 µl) containing 1 µg of HA vaccine prepared in the above-mentioned (1) and 2 µg of RCAI-8 was intranasally or subcutaneously administered for primary immunization. As the mouse, female BALB/c mice (CLEA Japan, Inc.) that became 6- to 8-week-old at the time of immunization were used in all experiments. All animal experiments were performed in NIID according to the animal experiment guide admitted by the Animal Protection Organization. As the positive control, synthetic double strand RNA poly(I:C) (obtained from TORAY INDUSTRIES, INC.) known to have an adjuvant activity was used. As the negative control, DMSO, solvent of RCAI-8, was used. Three weeks later, the mice were boostered in the same manner using or without using RCAI-8 or adjuvant. At 2 weeks from the final administration, each mouse was intranasally administered with 1000 PFU of A/PR8 virus suspended in PBS (1 µl) to allow infection of the upper respiratory tract. The series of procedures are shown in Fig. 1(A).

In addition, 5 mice in each experiment group were anesthetized with diethyl ether, 0.5% Tween 80-saline solution (5 µl) containing 1 µg of HA vaccine prepared in the above-mentioned (1) and 2 µg of RCAI-0 was intranasally or subcutaneously administered for primary immunization. As the mouse, female BALB/c mice (CLEA Japan, Inc.) that became 6- to 8-week-old at the time of immunization were used in all experiments. All animal experiments were performed in NIID according to the animal experiment guide admitted by the Animal Protection Organization. As the positive control, synthetic double strand RNA poly(I:C) (obtained from TORAY INDUSTRIES, INC.) known to have an adjuvant activity was used. As the negative control, DMSO, solvent of RCAI-0, was used. Three weeks later, the mice were boostered in the same manner using or without using RCAI-0 or adjuvant. At 2 weeks from the final administration, each mouse was intranasally administered with 1000 PFU of A/PR8 virus suspended in PBS (1 µl) to allow infection of the upper respiratory tract. The series of procedures are shown in Fig. 1(B).

### (4) Measurement of antibody titer

At 3 days after the virus infection, nasal lavage fluid and serum sample were recovered and the virus titer and the antibody titer were measured.

The mice were sacrificed under chloroform anesthesia, the serum and nasal lavage fluid were recovered and the virus titer and the antibody against A/PR8HA were measured. The levels of IgA and IgG antibodies against HA molecule purified from A/PR8 virus were measured by ELISA as reported in Tamura S, Ito Y, Asanuma H, Hirabayashi Y, Suzuki Y, Nagamine T, Aizawa C, Kurata T. Cross-protection against influenza virus infection afforded by trivalent inactivated vaccines inoculated intranasally with cholera toxin B subunit. J Immunol. 1992 Aug 1; 149(3):981-8.; Tamura S, Samegai Y, Kurata H, Nagamine T, Aizawa C, Kurata T. Protection against influenza virus infection by vaccine inoculated intranasally with cholera toxin B subunit. Vaccine. 1988 Oct; 6(5):409-13.; Tamura S, Yamanaka A, Shimohara M, Tomita T, Komase K, Tsuda Y, Suzuki Y, Nagamine T, Kawahara K, Danbara H, et al. Synergistic action of cholera toxin B subunit (and Escherichia coli heat-labile toxin B subunit) and a trace amount of cholera whole toxin as an adjuvant for nasal influenza vaccine. Vaccine. 1994 Apr;12(5):419-26. Specifically, the following components were used with a solid phase (EIA plate; Costar).
First component: HA molecule purified from A/PR8 virus according to the method of Phelan et al. (Phelan MA, Mayner RE, Bucher DJ, Ennis FA. Purification of influenza virus glycoproteins for the preparation and standardization of immunological potency testing reagents. J Biol Stand. 1980;8(3):233-42.)
Second component: nasal lavage fluid or serum
Third component: goat anti mouse IgA antibody (α chain specific; Amersham) or goat anti mouse IgG antibody (γ chain specific; Amersham) conjugated to biotin,
Fourth component: streptavidin (Life Technologies) conjugated to alkaline phosphatase
Fifth component: p-nitrophenylphosphate

Absorbance at 405 nm of produced chromogenic substrate was measured using an ELISA reader. As reported previously (Tamura S, Ito Y, Asanuma H, Hirabayashi Y, Suzuki Y, Nagamine T, Aizawa C, Kurata T. Cross-protection against influenza virus infection afforded by trivalent inactivated vaccines inoculated intranasally with cholera toxin B subunit. J Immunol. 1992 Aug 1; 149(3):981-8.; Tamura S, Samegai Y, Kurata H, Nagamine T, Aizawa C, Kurata T. Protection against influenza virus infection by vaccine inoculated intranasally with cholera toxin B subunit. Vaccine. 1988 Oct; 6(5):409-13.; Tamura S, Yamanaka A, Shimohara M, Tomita T, Komase K, Tsuda Y, Suzuki Y, Nagamine T, Kawahara K, Danbara H, et al. Synergistic action of cholera toxin B subunit (and Escherichia coli heat-labile toxin B subunit) and a trace amount of cholera whole toxin as an adjuvant for nasal influenza vaccine. Vaccine. 1994 Apr;12(5):419-26.), a 2-fold dilution series of purified HA specific IgA (320 ng/ml) or HA specific monoclonal IgG (160 ng/ml) was used as the standard.

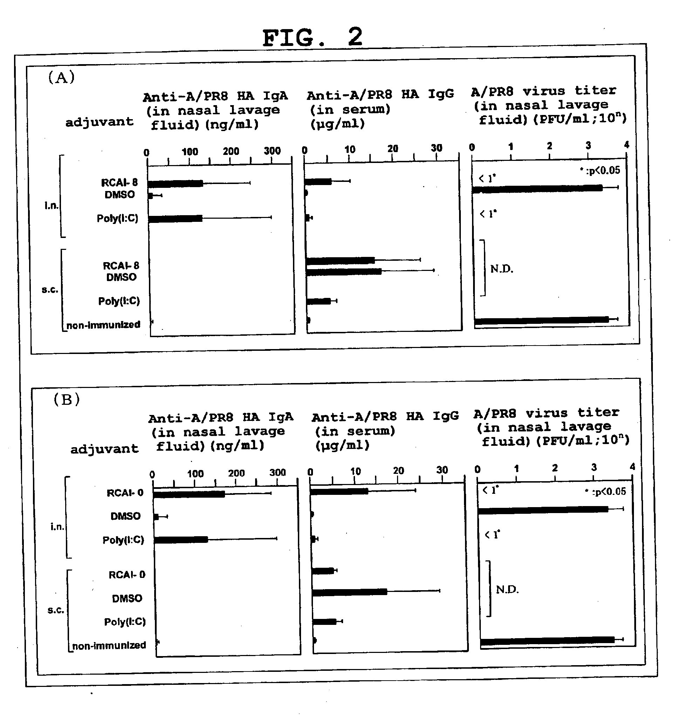

The results are shown in Fig. 2(A) (RCAI-8) and Fig. 2(B) (RCAI-0).

By intranasal administration of the compound having an NKT cell-activating action together with a virus antigen, remarkable decrease in an increase in intranasal antigen specific IgA, an increase in antigen specific IgG in serum, and the virus titer upon virus challenge was observed.

### Example 2 (Reference): Effect of the nasal vaccine of the present invention on avian influenza virus

The effect of the nasal vaccine of the present invention was examined using, as an antigen, H5N1 (A/Vietnam) virus, which was separated from an infection case occurred in Vietnam and inactivated (whole particles).

### (1) Preparation of vaccine

Using inactivated H5N1 virus as an antigen, whole virus particle vaccine was prepared. The whole virus particle vaccine was prepared by treating purified virus with 0.2% formalin. This virus was separated from human patients and can infect mammals. Similarly, it has acquired an ability to infect mouse.

### (2) Preparation of the compound having an NKT cell-activating action

As a compound having an NKT cell-activating action to be administered with virus antigen, RCAI-0 (prepared by a similar method as in Example 1 (2)) was used.

### (3) Administration of vaccine and virus challenge

Five mice in each experiment group were anesthetized with diethyl ether, 0.5% Tween 80-saline solution (10 µl) containing 1 µg of a vaccine prepared in the above-mentioned (1) and 2 µg of RCAI-0 was intranasally or subcutaneously administered for primary immunization. As the mouse, female BALB/c mice (CLEA Japan, Inc.) that became 6- to 8-week-old at the time of immunization were used in all experiments. All animal experiments were performed in NIID according to the animal experiment guide admitted by the Animal Protection Organization. As the positive control, synthetic double strand RNA poly(I:C) (obtained from TORAY INDUSTRIES, INC.) known to have an adjuvant activity was used. As the negative control, DMSO, solvent of RCAI-0, was used. Three weeks later, the mice were boostered in the same manner using or without using RCAI-0 or adjuvant. At 2 weeks from the final administration, each mouse was intranasally administered with 100 PFU of H5N1 virus suspended in PBS (2 µl) to cause infection of the upper respiratory tract.

### (4) Measurement of antibody titer

At 3 days after the virus infection, nasal lavage fluid and serum sample were recovered and the virus titer and the antibody titer were measured.

The mice were sacrificed under chloroform anesthesia, the serum and nasal lavage fluid were recovered and the virus titer and the antibody against H5N1 were measured. The levels of IgA and IgG antibodies against H5N1 vaccine antigen were measured by ELISA as in Example 1 (4). Specifically, the following components were used with a solid phase (EIA plate; Costar).
First component: H5N1 vaccine antigen
Second component: nasal lavage fluid or serum
Third component: goat anti mouse IgA antibody (α chain specific; Amersham) or goat anti mouse IgG antibody (γ chain specific; Amersham) conjugated to biotin,
Fourth component: streptavidin (Life Technologies) conjugated to alkaline phosphatase
Fifth component: p-nitrophenylphosphate

Absorbance at 405 nm of produced chromogenic substrate was measured using an ELISA reader. The serum and nasal cavity lavage fluid of the infected mouse were each used as the standard.

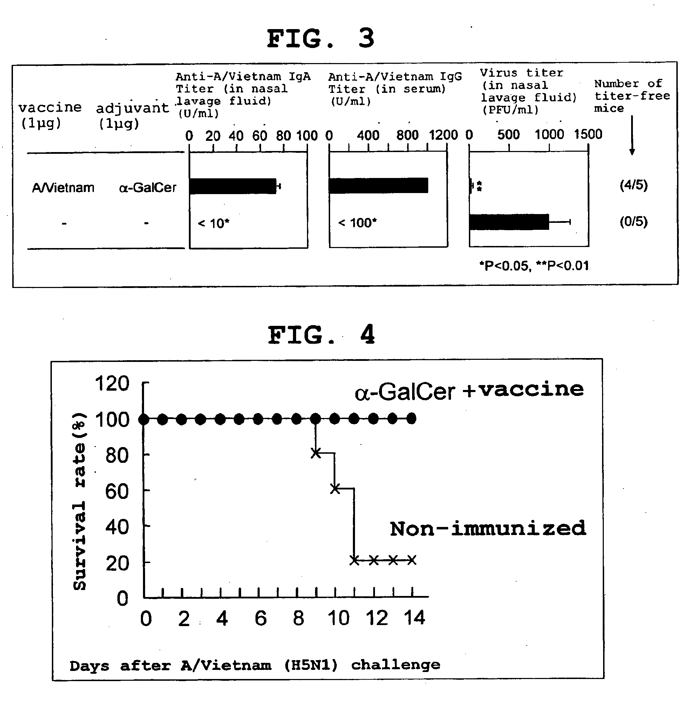

The results are shown in Fig. 3.

By intranasal administration of the compound having an NKT cell-activating action together with a virus antigen, the intranasal antibody titer increased significantly. In addition, the intranasal residual virus amount after virus infection became almost 0.

### (5) Evaluation of survival rate after virus challenge

PBS (20 µl) containing 100 PFU of H5N1 virus was intranasally inoculated to BALB/c mouse (non-immunized group (5 mice) and NKT ligand nasal vaccine (vaccine containing RCAI-0) vaccination group (5 mice)), the mice were observed once a day, and the survival rate of the both groups were examined. As a result, the non-immunized group showed a respiratory symptom as well as a neurological symptom and almost all mice died at day 11 from virus infection. The NKT ligand nasal vaccine group survived 100% even at day 14 from virus infection (Fig. 4).

### Industrial Applicability

By intranasal administration, the vaccine of the present invention can induce an increase in the antigen specific antibody titer in the upper respiratory tract, which is an entry pathway of many viruses and also an entry pathway of pollen antigens. Accordingly, the present invention enables more effective prophylaxis of virus infections and pollinosis. Moreover, the present invention is applicable to many virus infections, and is also applicable to H5 influenza virus (avian influenza virus) infection, which is currently the issue of concern all over the world.

This application is based on application Nos. 2005-106891 (filing date: April 1, 2005) and 2005-106894 (filing date: April 1, 2005) filed in Japan.

## Claims

1. A compound having an NKT cell-activating action represented by the following formula (I): 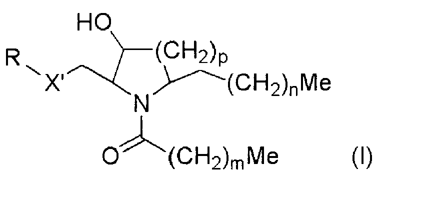 wherein R is an aldopyranose residue, X' is an oxygen atom, a sulfur atom or -NH-, m is an integer of 0 to 26, n is an integer of 0 to 16, p is an integer of 0 to 4, and Me is a methyl group, in combination with an immunogen in an amount effective for stimulating an immune response, for use as a nasal vaccine.

2. Compound for use according to claim 1, wherein the immunogen is derived from a pathogenic microorganism.

3. Compound for use according to claim 2, wherein the pathogenic microorganism is a virus.

4. Compound for use according to claim 2, wherein the pathogenic microorganism is at least one kind selected from the group consisting of influenza virus, avian influenza virus, severe acute respiratory syndrome (SARS) virus, acquired immunodeficiency syndrome (AIDS) virus and *Streptococcus pneumoniae.*

5. Compound for use according to claim 1, wherein the immunogen is derived from pollen.

6. Compound for use according to any one of claims 1 to 5, wherein R is α-D-galactopyranosyl.

7. Compound for use according to any one of claims 1 to 6, wherein p is 0 or 1.

8. Compound for use according to any one of claims 1 to 7, wherein n is 11 or 12.

9. Compound for use according to any one of claims 1 to 8, wherein m is 24.

10. Compound for use according to any one of claims 1 to 9, wherein X' is an oxygen atom.

11. A combination of (i) an immunogen in an amount effective for stimulating an immune response and (ii) an effective amount of a compound having an NKT cell-activating action represented by the following formula (I): 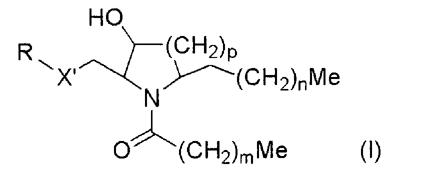 wherein R is an aldopyranose residue, X' is an oxygen atom, a sulfur atom or -NH-, m is an integer of 0 to 26, n is an integer of 0 to 16, p is an integer of 0 to 4, and Me is a methyl group, for use in inducing an intranasal immune response in a subject.

12. A combination for use according to claim 11, as further defined in any one of claims 3 to 10.

13. Use of a compound having an NKT cell-activating action represented by the following formula (I): 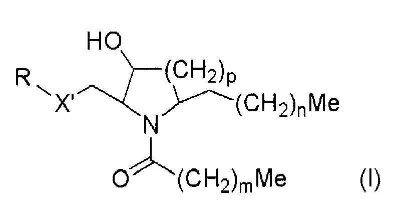 wherein R is an aldopyranose residue, X' is an oxygen atom, a sulfur atom or -NH-, m is an integer of 0 to 26, n is an integer of 0 to 16, p is an integer of 0 to 4, and Me is a methyl group, in combination with an immunogen,
for the production of a nasal vaccine.

14. Use according to claim 13, wherein the compound is as defined in any one of claims 6 to 10.

## Patentansprüche

1. Verbindung, die eine NKT-Zell-aktivierende Wirkung hat, die durch die folgende Formel (I) dargestellt wird: 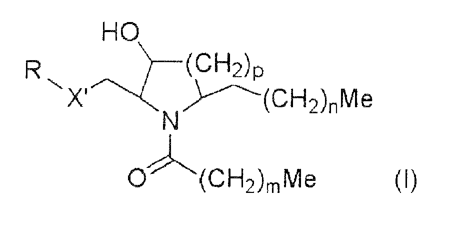 worin R ein Aldopyranose-Rest ist, X' ein Sauerstoffatom, ein Schwefelatom oder -NH- ist, m eine ganze Zahl von 0 bis 26 ist, n eine ganze Zahl von 0 bis 16 ist, p eine ganze Zahl von 0 bis 4 ist und Me eine Methylgruppe ist, in Kombination mit einem Immunogen in einer Menge, die zur Stimulierung einer Immunantwort wirksam ist, zur Verwendung als nasaler Impfstoff.

2. Verbindung zur Verwendung gemäß Anspruch 1, wobei das Immunogen von einem pathogenen Mikroorganismus stammt.

3. Verbindung zur Verwendung gemäß Anspruch 2, wobei der pathogene Mikroorganismus ein Virus ist.

4. Verbindung zur Verwendung gemäß Anspruch 2, wobei der pathogene Mikroorganismus wenigstens eine Art, ausgewählt aus der Gruppe, bestehend aus Influenzavirus, aviärem Influenzavirus, Virus des schweren akuten respiratorischen Syndroms (SARS), Virus des erworbenen Immundefizienzsyndroms (AIDS) und Streptococcus pneumoniae, ist.

5. Verbindung zur Verwendung gemäß Anspruch 1, wobei das Immunogen aus Pollen stammt.

6. Verbindung zur Verwendung gemäß einem der Ansprüche 1 bis 5, wobei R α-D-Galactopyranosyl ist.

7. Verbindung zur Verwendung gemäß einem der Ansprüche 1 bis 6, wobei p 0 oder 1 ist.

8. Verbindung zur Verwendung gemäß einem der Ansprüche 1 bis 7, wobei n 11 oder 12 ist.

9. Verbindung zur Verwendung gemäß einem der Ansprüche 1 bis 8, wobei m 24 ist.

10. Verbindung zur Verwendung gemäß einem der Ansprüche 1 bis 9, wobei X' ein Sauerstoffatom ist.

11. Kombination aus (i) einem Immunogen in einer Menge, die zur Stimulierung einer Immunantwort wirksam ist, und (ii) einer wirksamen Menge einer Verbindung, die eine NKT-Zell-aktivierende Wirkung hat, die durch die folgende Formel (I) dargestellt wird: 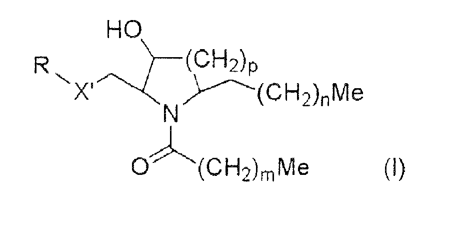 worin R ein Aldopyranose-Rest ist, X' ein Sauerstoffatom, ein Schwefelatom oder -NH- ist, m eine ganze Zahl von 0 bis 26 ist, n eine ganze Zahl von 0 bis 16 ist, p eine ganze Zahl von 0 bis 4 ist und Me eine Methylgruppe ist, zur Verwendung beim Induzieren einer intranasalen Immunantwort bei einem Subjekt.

12. Kombination zur Verwendung gemäß Anspruch 11, wie sie außerdem in einem der Ansprüche 3 bis 10 definiert ist.

13. Verwendung einer Verbindung, die eine NKT-Zell-aktivierende Wirkung hat, die durch die folgende Formel (I) dargestellt wird: 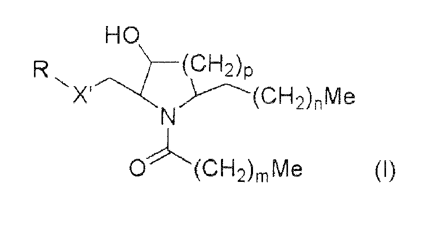 worin R ein Aldopyranose-Rest ist, X' ein Sauerstoffatom, ein Schwefelatom oder -NH- ist, m eine ganze Zahl von 0 bis 26 ist, n eine ganze Zahl von 0 bis 16 ist, p eine ganze Zahl von 0 bis 4 und Me eine Methylgruppe ist, in Kombination mit einem Immunogen zur Herstellung eines nasalen Impfstoffs.

14. Verwendung gemäß Anspruch 13, wobei die Verbindung wie in einem der Ansprüche 6 bis 10 definiert ist.

## Revendications

1. Composé doté d'un effet d'activation des lymphocytes NKT, représenté par la formule (I) suivante : 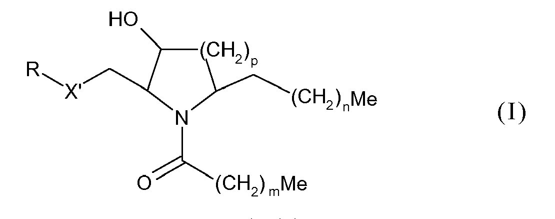 dans laquelle R représente un reste d'aldopyranose, X' représente un atome d'oxygène ou de soufre ou un chaînon symbolisé par -NH-, l'indice m est un nombre entier valant de 0 à 26, l'indice n est un nombre entier valant de 0 à 16, l'indice p est un nombre entier valant de 0 à 4, et Me représente un groupe méthyle,
en combinaison avec un agent immunogène présent en une quantité à effet de stimulation d'une réponse immune, pour utilisation en tant que vaccin nasal.

2. Composé pour utilisation conforme à la revendication 1, pour lequel l'agent immunogène dérive d'un microorganisme pathogène.

3. Composé pour utilisation conforme à la revendication 2, pour lequel le microorganisme pathogène est un virus.

4. Composé pour utilisation conforme à la revendication 2, pour lequel le microorganisme pathogène est d'au moins une sorte, choisie dans l'ensemble constitué par un virus de grippe, un virus de grippe aviaire, un virus de syndrome respiratoire aigu sévère (SRAS), un virus de syndrome d'immunodéficience acquise (SIDA), et une bactérie *Streptococcus pneumoniae.*

5. Composé pour utilisation conforme à la revendication 1, pour lequel l'agent immunogène dérive d'un pollen.

6. Composé pour utilisation conforme à l'une des revendications 1 à 5, dans lequel R représente un reste α-D-galactopyranosyle.

7. Composé pour utilisation conforme à l'une des revendications 1 à 6, dans lequel l'indice p vaut 0 ou 1.

8. Composé pour utilisation conforme à l'une des revendications 1 à 7, dans lequel l'indice n vaut 11 ou 12.

9. Composé pour utilisation conforme à l'une des revendications 1 à 8, dans lequel l'indice m vaut 24.

10. Composé pour utilisation conforme à l'une des revendications 1 à 9, dans lequel X' représente un atome d'oxygène.

11. Combinaison
i) d'un agent immunogène, en une quantité à effet de stimulation d'une réponse immune,
ii) et, en une quantité efficace, d'un composé doté d'un effet d'activation des lymphocytes NKT, représenté par la formule (I) suivante : 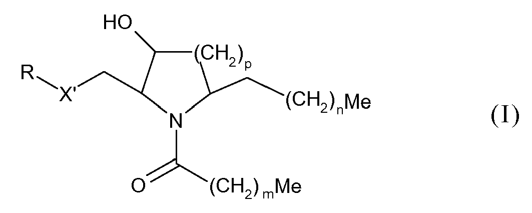
dans laquelle R représente un reste d'aldopyranose, X' représente
un atome d'oxygène ou de soufre ou un chaînon symbolisé par -NH-, l'indice m est un nombre entier valant de 0 à 26, l'indice n est un nombre entier valant de 0 à 16, l'indice p est un nombre entier valant de 0 à 4, et Me représente un groupe méthyle,
pour utilisation afin d'induire une réponse immune intra-nasale chez un sujet.

12. Combinaison pour utilisation, conforme à la revendication 11, telle que définie plus précisément dans l'une des revendications 3 à 10.

13. Utilisation d'un composé doté d'un effet d'activation des lymphocytes NKT, représenté par la formule (I) suivante : 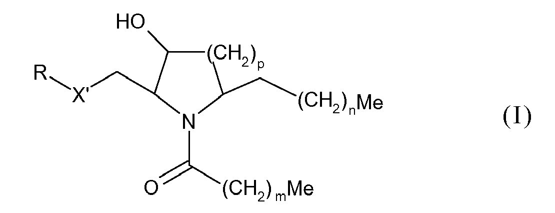 dans laquelle R représente un reste d'aldopyranose, X' représente un atome d'oxygène ou de soufre ou un chaînon symbolisé par -NH-, l'indice m est un nombre entier valant de 0 à 26, l'indice n est un nombre entier valant de 0 à 16, l'indice p est un nombre entier valant de 0 à 4, et Me représente un groupe méthyle,
en combinaison avec un agent immunogène, afin de produire un vaccin nasal.

14. Utilisation conforme à la revendication 13, pour laquelle le composé est tel que défini dans l'une des revendications 6 à 10.
